# EUROPEAN PATENT SPECIFICATION

(11) **EP 4 222 493 B1**
(45) Date of publication and mention of the grant of the patent: **30.04.2025**
(21) Application number: 21794089.9
(22) Date of filing: 30.09.2021
(51) Int. Cl.: G01N 33/50, G01N 33/68

(54) **SCREENING TEST FOR CATSPER MODULATORS**
SCREENING-TEST FÜR CATSPER-MODULATOREN
TEST DE DÉPISTAGE POUR MODULATEURS DE CATSPER

(30) Priority: 30.09.2020 LU 102104
(43) Date of publication of application: 09.08.2023
(73) Proprietor: Westfälische Wilhelms-Universität Münster, 48149 Münster (DE)
(72) Inventor: STRÜNKER, Timo, 50937 Köln (DE); SCHIFFER, Christian, 41516 Grevenbroich (DE); BRENKER, Christoph, 48159 Münster (DE); YOUNG, Samuel Zachary, 48149 Münster (DE)
(74) Representative: Schiweck Weinzierl Koch Patentanwälte Partnerschaft mbB
(86) International application number: PCT/EP2021/076908
(87) International publication number: WO 2022/069611

(56) References cited:
- WO-A1-2020/193446
- US-A1- 2015 335 622
- TORRES-FLORES V. ET AL: "Sodium influx induced by external calcium chelation decreases human sperm motility", vol. 26, no. 10, 1 October 2011 (2011-10-01), GB, pages 2626 - 2635, XP055798290, ISSN: 0268-1161, Retrieved from the Internet <URL:https://www.ncbi.nlm.nih.gov/pmc/articles/PMC3174032/pdf/der237.pdf> DOI: 10.1093/humrep/der237
- CARLSON ANNE E. ET AL: "Pharmacological Targeting of Native CatSper Channels Reveals a Required Role in Maintenance of Sperm Hyperactivation", vol. 4, no. 8, 1 January 2009 (2009-01-01), pages e6844, XP055798413, Retrieved from the Internet <URL:https://storage.googleapis.com/plos-corpus-prod/10.1371/journal.pone.0006844/1/pone.0006844.pdf?X-Goog-Algorithm=GOOG4-RSA-SHA256&X-Goog-Credential=wombat-sa@plos-prod.iam.gserviceaccount.com/20210423/auto/storage/goog4_request&X-Goog-Date=20210423T100832Z&X-Goog-Expires=3600&X-Goog-SignedHeaders=ho> DOI: 10.1371/journal.pone.0006844
- MARTINS DA SILVA SARAH J. ET AL: "Drug discovery for male subfertility using high-throughput screening: a new approach to an unsolved problem", HUMAN REPRODUCTION, vol. 32, no. 5, 16 March 2017 (2017-03-16), GB, pages 974 - 984, XP055797962, ISSN: 0268-1161, DOI: 10.1093/humrep/dex055

## Description

### FIELD OF THE INVENTION

The present invention relates to a method of identifying a modulator of the CatSper channel activity, comprising the steps of: a) contacting a sperm sample with a test solution that is essentially free of free calcium and a test compound; b) detecting the motility of each sperm cell in the sample under the test condition of step (a); and c) identifying the test compound as a modulator of CatSper channel activity if the presence of the compound is correlated with an altered motility of the sperm cell relative to the motility of the sperm cell in the test solution without the test compound, as defined in the appended claims.

### BACKGROUND OF THE INVENTION

In the desire to prevent unwanted pregnancies despite sexual intercourse people have used contraceptive methods (contraception) since ancient times. Today a number of different options exist with different applications and reliabilities. The "contraceptive pill", first approved in the early 1960s, enjoys particular popularity but has two decisive disadvantages: first, various side effects can occur due to a change in the natural hormone balance; second, the burden of contraception must be borne exclusively by women.

For this reason, intensive research is currently conducted to find non-hormonal alternatives. A concept for non-hormonal contraception is based on a destroyed sperm function such that it is impossible for the sperm to fertilize an egg.

The sperm-specific CatSper ion channel controls the intracellular calcium concentration of sperm and, thereby, the swimming behavior. In human sperm, CatSper is activated by progesterone, a female sex hormone that is secreted by the egg to assist the sperm in navigating inside the oviduct and in fertilizing the egg. Progesterone-induced CatSper activation results in a calcium influx that triggers changes in the flagellar beating pattern and sperm motility. In the immediate vicinity of the egg, the CatSper activation leads to so-called "hyperactive" swimming of the sperm that is required to penetrate the egg coat. Without CatSper, sperm fail to hyperactivate and to penetrate the egg coat and, thus, to fertilize the egg. Genetic aberrations in genes encoding CatSper-channel subunits that result in a loss of CatSper function cause male infertility. From these results, it was concluded that inhibition of CatSper might be used to pharmacologically prevent fertilization.

Another important finding reinforces that CatSper represents a promising target for novel contraceptives. The CatSper channel is exclusively expressed in sperm, which makes the channel particularly promising in terms of the development of a contraceptive with no or only few side effects. In theory, it would be possible to develop a crème, a lubricant gel or even a drug that can be taken by men ("the pill for men") and which eliminates or disturbs the CatSper channel activity by either inhibition or premature activation of the channel already in the male body, rendering the sperm unable to fertilize; alternatively, such a contraceptive could be used by women for the same purpose.

In contrast to the potential role as a contraceptive target described above, CatSper could be pharmacologically targeted to support the sperm cell motility and, thereby, increase male fertility. Here, CatSper activators could likewise be taken by men diagnosed with impaired sperm motility.

The decisive step in the development of such a medicament is the systematic research for active compounds that modulate (activate or inhibit) the CatSper channel activity.

Currently, the effect of pharmacological compounds on CatSper is either examined directly by measuring the ion flow through the channel pore (calcium fluorimetry or electrophysiology) or indirectly by observing subtle sperm motility changes, especially observing hyperactivation of the sperm. To integrate these currently available methods into a high-throughput drug screening process is practically impossible due to the immense costs for infrastructure, consumable supplies or the read-out of CatSper-modulation, which is difficult to detect and timely limited. It is thus too complicated and laborious to use the prior art for drug-screening purposes.

US2015/0335622 discloses antagonists of the CatSper1 channel activity for inhibiting sperm hyperactivity and their use as contraceptive agents. CatSper1 inhibitors are screened in a sperm motility assay with a test solution that is not essentially free of free calcium.

There remains a need in the prior art to provide a high-throughput screening method for CatSper modulators. The technical problem underlying the present application is thus to comply with this need. The technical problem is solved by providing the embodiments reflected in the claims, described in the description and illustrated in the examples and figures that follow.

### SUMMARY OF THE INVENTION

The present inventors have developed a novel, fundamentally simpler assay compared to fluorescence optical / electrophysiological methods or calcium fluorimetry to screen for modulators of the CatSper channel, which can be used for a reliable prediction of test compounds usable in contraception or male fertility promotion.

In particular, the present inventors have found an *in vitro* screening assay, which is safe, reliable and easy to handle, which determines modulators of the CatSper channel in a high throughput manner. The simplicity of the method of the present invention is demonstrated in that it can be performed directly with the native ejaculate, thus eliminating the need for preceding labor-intensive sperm purification. Further, no specific devices are needed to use in order to identify modulators of the CatSper ion channel according to the present invention. This screening is interpretable due to the experimental conditions, which are solutions essentially free of free-calcium. This enables a clear distinction between motile and immotile sperm cells and the identification of CatSper modulators. Finally, the amount of work for the analysis of the motility of the spermatozoa itself is very limited. Thus, by applying the assay of the present invention, the outcome or results whether a compound is capable of modulating the CatSper ion channel is very quickly available. A particular goal is thus to use the screening test for the identification of modulators of the CatSper channel for the development of new, non-hormonal contraceptives or for male fertility promotion.

In sum, the present invention enables a substantial optimization and rationalization of the "drug-discovery" process that identifies CatSper modulators. The screening test is, in short, currently the only method that is suitable for a high-throughput screening on an industrial scale.

Accordingly, in a first aspect, the present invention relates to a method of identifying a modulator of CatSper channel activity, comprising the steps of: a) contacting a sperm sample with a test solution that is essentially free of free calcium, wherein the test solution that is essentially free of free calcium comprises less than about 20 nM free calcium, and a test compound; b) detecting the motility of each sperm cell in the sample under the test condition of step (a); c) identifying the test compound as a modulator of CatSper channel activity if the presence of the compound is correlated with an altered motility of the sperm cell relative to the motility of the sperm cell in the test solution without the test compound.

Further envisaged herein is the method as defined above, wherein the sperm sample may be contacted for at least about 5 minutes to at most about 30 minutes with the test solution and the test compound in step (a), preferably the sample is contacted for about 15 minutes.

The present invention may also disclose the method as defined elsewhere herein, wherein if the presence of the test compound is correlated with a decreased motility of the sperm cell relative to the motility of the sperm cell in the test solution without the test compound, it may be indicative that the test compound is a modulator capable of activating CatSper channel activity.

Encompassed herein is also the method as defined elsewhere herein, wherein if the presence of the compound is correlated with an increased motility of the sperm cell relative to the motility of the sperm cell in the test solution without the test compound, it may be indicative that the test compound is a modulator capable of inhibiting CatSper channel activity.

Also described herein is the method as defined elsewhere herein, wherein if the presence of the compound is correlated with no difference in the motility of the sperm cell relative to the motility of the sperm cell in the test solution without the test compound, it may be indicative that the test compound is not a modulator of CatSper channel activity.

In the present invention, the test solution that is essentially free of free calcium comprises less than about 20 nM free calcium. Further, the test solution of the method as described elsewhere herein, which is essentially free of free calcium, may comprise at least one agent reducing the free calcium concentration that is a chemical capable of binding Ca²⁺. Preferably, the chemical capable of binding Ca²⁺ used in the method as defined elsewhere herein to reduce the free calcium concentration is any one of EGTA, EDTA, BAPTA, HEDTA, NTA, DiBrBAPTA, citric acid, gluconate solution, aspartate solution, phosphate, or a Ca²⁺-indicator dye, preferably EGTA.

The present invention may also disclose the method as defined elsewhere herein, wherein the test solution, which is essentially free of free calcium, may further be essentially free of any free divalent ions. Preferably, the test solution that is essentially free of free calcium being used in the method defined elsewhere herein is further essentially free of free magnesium, wherein the test solution that is essentially free of free calcium and that is further essentially free of free magnesium being used in the method defined elsewhere herein comprises less than about 250 µM free magnesium.

It may also be comprised by the method described elsewhere herein, wherein the test compound may be any one of a ligand, a chemical compound, an aptamer, or a lipid or a combination thereof. Preferably, the ligand is any one of a chemical compound, a peptide, or a protein or a combination thereof.

The method of the present invention may also envisage that the sperm sample may be contacted simultaneously with the test solution and the test compound.

It may also be encompassed by the method as defined elsewhere herein that the motility of each sperm cell may be characterized by any forward movement in comparison to immotility of a sperm cell characterized by no forward movement. Further, the present invention may also comprise the method as defined elsewhere herein, wherein the motility of each sperm cell may be determined by using any method selected from the group consisting of light microscopy, differential-dynamic microscopy, videomicrography, and methods detecting light-scattering. Preferably, the motility of each sperm cell is determined using light microscopy.

The present invention may also comprise the method as defined elsewhere herein, wherein the motility of each sperm cell may be determined manually or automatically. It is further encompassed by the method of the present invention that manually determining the motility of each sperm cell may comprise counting of at least about 10 sperm cells of the sperm sample under the conditions of step (b).

The present invention may also comprise the method as defined elsewhere herein, wherein the sperm sample may be obtained from a male subject, preferably a vertebrate, more preferably a mammal. Preferably, the mammal from which the sample is obtained for the method as defined elsewhere herein is any one of a human, a cow, a buffalo, a horse, a donkey, a camel, a sheep, a goat, a pig, a dog, a cat, a rat, or a mouse, preferably a human.

### BRIEF DESCRIPTION OF THE FIGURES

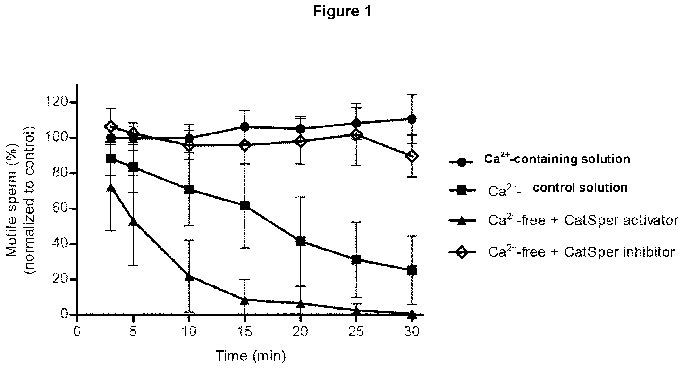

**Fig. 1****: Time course of sperm motility in the CatSper modulation test.** Ca²⁺⁻free control solution = Ca²⁺⁻free solution without the test compound; CatSper activator = progesterone (Strünker et al. Nature 471(7338), 382-386, 2011); CatSper inhibitor = RU1968 (Rennhack et al., British journal of pharmacology 175, 3144-3161, 2018).

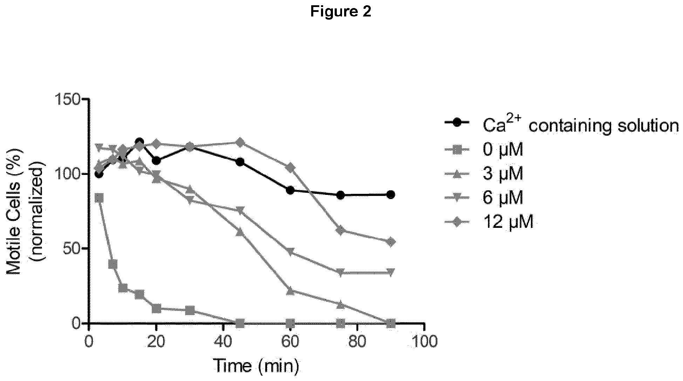

**Fig. 2****: The CatSper inhibitor modulates the sperm cell motility in a concentration-dependent manner under essentially Ca²⁺-free conditions.** Sperm cells were exposed to a calcium-free buffer (grey curves) containing different concentrations of the exemplified CatSper inhibitor RU1968 at time t = 0 minutes up to t = 90 minutes. A control sample (black, HTF) was kept under Ca²⁺-containing conditions. During a subsequent incubation at 37 °C, the motility in the sperm population was determined by light microscopy; the data was normalized (first reading in the Ca²⁺-containing control = 100%).

### DETAILED DESCRIPTION OF THE INVENTION

In order to overcome some of the shortcomings for contraception of the means described so far in the prior art, the inventors provide herein a promising new method of identifying modulators of the CatSper ion channel activity based on detecting the motility of sperm cells obtained from a male subject, wherein calcium-free test conditions are used. In particular, the inventors of the present invention developed a high-throughput method for identifying modulators of the CatSper ion channel, which can then be used for contraception or male fertility promotion. In sum, the present invention opens a new avenue for the industry of the development of hormone-free contraception's and male fertility products.

As described above, the present invention relates to a method of identifying a modulator of CatSper channel activity, comprising the steps of: a) contacting a sperm sample preferably obtained from a male subject with a test solution that is essentially free of free calcium, wherein the test solution that is essentially free of free calcium comprises less than about 20 nM free calcium, and a test compound; b) detecting the motility of each sperm cell in the sample under the test condition of step (a); c) identifying the test compound as a modulator of CatSper channel activity if the presence of the compound is correlated with an altered motility of the sperm cell relative to the motility of the sperm cell in the test solution without the test compound.

The method of the present invention is thus *in vitro.* The term "*in vitro*" refers in this case to *ex vivo.*

As used herein and throughout the present invention, the term "CatSper ion channel" or "CatSper channel" refers to a sperm-specific *cation channel of spermatozoa* Ca²⁺-channel, which is located in the cell membrane of a spermatozoa flagellum. The CatSper channel complex comprises four homologous α subunits (CatSper 1-4) and at least six auxiliary subunits: CatSper β, CatSper γ, CatSper δ, CatSper ε and CatSper 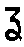 , and EFCAB9

A "modulator" of said CatSper channel / of CatSper channel activity refers to an organic or inorganic test compound being capable of inhibiting or activating the CatSper ion channel activity. Thus, a modulator of the CatSper ion channel refers according to the present invention to an inhibitor or an activator thereof. An inhibitor is capable of inhibiting the activity of something (e.g. inhibits the CatSper channel activity). In this context and as used throughout the present invention, the term "inhibiting / inhibit the CatSper channel activity" means that a smaller number of ions are flowing per unit time through the pore of the CatSper channel at a given membrane potential in comparison to the number of ions flowing per unit time through the pore of the CatSper channel at a given membrane potential without being inhibited by any modulator. The term "inhibit" or "inhibiting" the CatSper channel activity can also refer to decrease / decreasing the CatSper channel activity.

An activator is a modulator that is capable of activating the activity of something (e.g. activates the CatSper channel activity). In this context and as used throughout the present invention, the term "activating / activate the CatSper channel activity" means that a larger number of ions are flowing per unit time through the pore of the CatSper channel at a given membrane potential in comparison to the number of ions flowing per unit time through the pore of the CatSper channel at a given membrane potential without being activated by any modulator. The term "activate" or "activating" the CatSper channel activity can also refer to increase / increasing the CatSper channel activity.

### Step a) of the method of the present invention

The method of the present invention can be applied to untreated semen or sperm which is directly sampled from fresh ejaculate. In other words, an untreated sperm sample may be used in the method of the present invention. However, it can be advantageous to incubate the semen or sperm (sperm sample) before applying the method of the present invention. An optional incubation at 37 °C for about 30 minutes may decrease the viscosity of the sperm and eases the general handling, where necessary.

When the method of the present invention is applied, first a sperm sample obtained from a male subject is contacted with a test solution that is essentially free of free calcium and with a test compound, which refers to step (a) of the method of identifying modulators of the CatSper ion channel activity according to the present invention. The test solution being essentially free of free calcium also refers to a "calcium-free test solution" or simply refers to the "test solution". When the test solution as defined herein, but no test compound is contacted with the sperm sample this refers to the control solution when used herein. Thus, when a definition herein (except for the addition of the test compound) refers to the test solution it may also refer to the control solution mentioned herein.

The molecular composition of the test solution and the control solution are thereby almost identical; they differ only in the presence or absence of the test compound. In other words, the test solution comprises the test compound and is essentially free of free-calcium, and the control solution lacks the test compound and is essentially free of free-calcium. Said solutions (test and control) being essentially free of free calcium have the same composition as the calcium-free solution listed in **Table 1** of Example 1.

The test solution and control solution both being "essentially free of free calcium" according to the method of the present invention comprise less than about 20 nM, preferably less than about 15 nM, more preferably less than about 10 nM, more preferably less than about 5 nM, such as less than about 19 nM, less than about 18 nM, less than about 17 nM, less than about 16 nM, less than about 15 nM, less than about 14 nM, less than about 13 nM, less than about 12 nM, less than about 11 nM, less than about 10 nM, less than about 9 nM, less than about 8 nM, less than about 7 nM, less than about 6 nM, less than about 5 nM, less than about 4 nM, less than about 3 nM, less than about 2 nM, or less than about 1 nM free calcium. Accordingly, a solution comprising less than about 20 nM, less than about 15 nM, less than about 10 nM, or less than about 5 nM, such as about 19 nM, about 18 nM, about 17 nM, about 16 nM, about 15 nM, about 14 nM, about 13 nM, about 12 nM, about 11 nM, about 10 nM, about 9 nM, about 8 nM, about 7 nM, about 6 nM, about 5 nM, about 4 nM, about 3 nM, about 2 nM, or about 1 nM free calcium qualifies as a test solution or a control solution being essentially free of free calcium according to the present invention. As mentioned elsewhere herein, said test solution (and control solution) according to the present invention comprises less than about 20 nM of free calcium and is thus called "essentially free of free calcium" or "calcium-free".

As used herein, the term "free calcium" refers to calcium not being bound to proteins such as albumin and/or globulin or not being (complex-) bound to for example bicarbonate, lactate, citrate, phosphate and/or a chelator. In this context and as used herein, the term "calcium" means Ca²⁺. Detection methods for detecting / measuring free calcium are known to the person skilled in the art. Preferably, any ion-selective electrode measurement detecting only free calcium can be used.

The "test solution being essentially free of free calcium" being used in the present invention may comprise at least one agent reducing the free calcium concentration. The term "free calcium reducing agent" may also be used interchangeably with the term "agent reducing the free calcium concentration". Under physiological conditions, the calcium concentration within a sperm cell is about 10.000 lower compared to the extracellular fluid - it is on the order of about 200-500 nM versus about 2 mM. Along this concentration gradient, the CatSper ion channel may mediate an influx of calcium into the sperm cell. While calcium pumps or calcium exchangers in the sperm cell membrane may export calcium ions to the outside, the calcium influx may be compensated and calcium homeostasis may be maintained. By reversing the calcium flux through the CatSper ion channel (by lowering the free extracellular calcium concentration with at least one agent reducing the free calcium concentration below the intracellular concentration), the intracellular calcium concentration may decrease as Ca²⁺ ions flow out of the sperm cell through the CatSper ion channel. This decrease in the intracellular calcium concentration is not tolerated by the sperm cells, i.e. they become immotile. Thus, with at least one agent being capable of reducing the free calcium concentration, the extracellular calcium concentration may be reduced below the intracellular calcium concentration in the spermatozoon. Therefore, by reducing the free calcium concentration it is meant that the free calcium concentration of a solution is lowered to, or less than about 20 nM of free calcium as defined above, thereby providing a "test solution being essentially free of free calcium" described elsewhere herein.

Preferably, the at least one agent reducing the free calcium concentration is a chemical binding Ca²⁺. In this context and as used throughout the description, the term "chemical binding Ca²⁺" is any substance or molecule with a distinct molecular composition that is produced by, or used in a chemical process and which is able to bind free calcium. More preferably, said chemical binding Ca²⁺ used in the present invention is any one of a chelator, citric acid, gluconate solution, aspartate solution, phosphate or a Ca²⁺-indicator dye, preferably a chelator. Even more preferably, said chemical binding Ca²⁺ used in the present invention is any one of ethylene glycol-bis(β-aminoethyl ether)-*N*,*N*,*N'*,*N'*-tetraacetic acid (short: EGTA), ethylenediaminetetraacetic acid (short: EDTA), 1,2-bis(*o*-aminophenoxy)ethane-*N*,*N*,*N'*,*N'*-tetraacetic acid (short: BAPTA), *N*-(2-Hydroxyethyl)ethylenediamine-*N*,*N'*,*N'*-triacetic acid (short: HEDTA), Nitrilotriacetic acid (short: NTA), dibromo-1,2,-bis(o-aminophenoxy)ethane-N,N,-N',N',tetraacetic acid (short: DiBrBAPTA), citric acid, gluconate solution, aspartate solution, phosphate, or a Ca²⁺-indicator dye, most preferably EGTA. Said chemical binding Ca²⁺ may also be any one of EGTA, EDTA, BAPTA, HEDTA, NTA, DiBrBAPTA, citric acid, gluconate solution, aspartate solution, phosphate, or a Ca²⁺-indicator dye, preferably EGTA or any derivative of EGTA, EDTA, BAPTA, HEDTA, NTA, DiBrBAPTA, citric acid, gluconate solution, aspartate solution, or phosphate.

EGTA, EDTA, BAPTA, HEDTA, NTA, DiBrBAPTA or derivatives thereof being mentioned herein as Ca²⁺-binding agents refer to chelators. A "chelator" is any organic or inorganic compound having two or more ion pairs and, thus, capable of forming more than one coordinate bond with a central (metal)-ion. In fact, said chelators coordinate divalent (metal) cations with high affinity and make them unavailable for ion channels. Thus, such chelators may have the ability to fix divalent or polyvalent cations in stable complexes (so-called chelates). If any one of the chelators as mentioned elsewhere herein may be used as a chemical binding Ca²⁺, at least about 2.5 mM, at least about 3 mM, at least about 3.5 mM, at least about 4 mM, at least about 4.5 mM, at least about 5 mM, at least about 5.5 mM, at least about 6 mM, at least about 6.5 mM, at least about 7 mM, at least about 7.5 mM, at least about 8 mM, at least about 8.5 mM, at least about 9 mM, at least about 9.5 mM, or at least about 10mM, preferably about 2.5 mM, about 3 mM, about 3.5 mM, about 4 mM, about 4.5 mM, about 5 mM, about 5.5 mM, about 6 mM, about 6.5 mM, about 7 mM, about 7.5 mM, about 8 mM, about 8.5 mM, about 9 mM, about 9.5 mM, about 10 mM of said chelator may be used according to the methods of the present invention. More preferably, any number in the range between about 2.5 mM to about 10 mM, between about 2.9 mM to about 8.8 mM, between about 3.3 mM to about 7.5 mM, or between about 4 mM to about 6 mM of said chelator may be used in the calcium-free test and control solution according to the methods of the present invention. Most preferably, about 5 mM EGTA are used as chemical binding Ca²⁺ in the calcium-free test solution according to the present invention.

Preferably, the test and control solution that are essentially free of free calcium are further essentially free of any free divalent ions. "Free divalent ions" as used herein and throughout the present invention refers besides Ca²⁺ to ions or cations such as Mg²⁺, Cu²⁺ or Fe²⁺. More preferably, the test and control solution, which are essentially free of free calcium, are further essentially free of free magnesium (Mg²⁺). Even more preferably, the test and control solution, which are essentially free of free calcium and are further essentially free of free magnesium comprise less than about 250 µM of free magnesium. In other words, the concentration of free magnesium in the test and control solution may be less than about 250 µM. Higher concentrations of magnesium (Mg²⁺) inhibit CatSper activity and the assay would then therefore no longer be feasible. This disclosure also includes in particular less than about 250 µM, less than about 240 µM, less than about 230 µM, less than about 220 µM, less than about 210 µM, less than about 200 µM, less than about 190 µM, less than about 180 µM, less than about 170 µM, less than about 160 µM, less than about 150 µM, less than about 140 µM, less than about 130 µM, less than about 120 µM, less than about 110 µM, or less than about 100 µM. Most preferably, the test and control solution which are essentially free of free calcium and are further essentially free of free magnesium comprise about 200 µM free magnesium. Alternatively, it may also be comprised herein that the test and control solution, which are essentially free of free calcium comprise no free magnesium (such as about 0 µM magnesium).

The "test compound" used in the present invention may be any compound known in the art which can be tested in the method of the present invention, whether said test compound is a modulator of CatSper channel activity as defined elsewhere herein or whether said test compound is not a modulator of CatSper channel activity. The test compound used in the present invention is preferably any one of a ligand, a chemical compound, an aptamer, or a lipid or a combination thereof.

A "ligand" as used herein and throughout the present invention refers to a small organic molecule, a protein, a peptide that directly binds to said CatSper channel or that binds to a receptor protein that affects the activity of the CatSper channel. Thus, by binding to said channel or receptor protein, the "ligand" is able to activate or inhibit said channel activity. Preferably, the ligand activating or inhibiting the CatSper channel activity is any one of a chemical compound, a peptide, or a protein or a combination thereof. In this context, a "protein" includes, but is not limited to, an antibody or a lipocalin known to the person skilled in the art.

As used herein and throughout the description a "chemical compound" refers to a chemical substance composed of many identical molecules or molecular entities composed of atoms from more than one element held together by chemical bonds. If the test compound being used in the method of the present invention is a lipid, it could be any one of a steroid or a prostaglandin known to the skilled person.

The test compound used in the present invention however is not RU1968, which is an already known inhibitor of the CatSper channel (Rennhack et al., British journal of pharmacology 175, 3144-3161, 2018). Said specific RU1968 inhibitor may pharmacologically mimic the phenotype of a CatSper-deficiency, meaning when applying the inhibitor RU1968, this may correspond to the same effect as if a male subject suffers from absence or a functional defect of the CatSper channel. The same applies for progesterone, which is an already known activator of the CatSper channel. Thus, the test compound used in the present invention however is not progesterone. Applying RU1968 as an example of an inhibitor of CatSper channel activity or progesterone as an example of an activator of CatSper channel activity demonstrates that the screening test of the present invention is suitable as a phenotypic screening method for testing test compounds as described elsewhere herein in search of CatSper modulators.

In this context and as used throughout the present invention, the term "contacted"/ "contacting with" means adding the sperm cells (comprised in a sperm sample being obtained from a male subject) to the test solution and the test compound as described elsewhere herein or adding the test solution and the test compound as described elsewhere herein to the sperm cells (comprised in a sperm sample being obtained from a male subject). In this context and as used throughout the present invention, it means that the sperm sample is either contacted with the test solution and the test compound separately (meaning the test solution and the test compound are separately added into a tube, where the sperm sample is already in the same tube) or the sperm sample is contacted simultaneously with the test solution and the test compound (meaning 1) the test solution already comprising the test compound is added into a tube, where the sperm sample is already in the same tube, or 2.) the test solution and the test compound (also the test solution comprising the test compound) are added into a tube, and then the sperm sample is added into the same tube). Preferably, the sperm sample is contacted simultaneously (at the same time) with the test solution and the test compound as defined elsewhere herein. Preferably, at least about 0.1 µl and/or at most about 20 µl, such as about 0.1, 0.5, 1, 2, 3, 4, 5, 6, 7, 8, 9, 10, 11, 12, 13, 14, 15, 16, 17, 18, 19 or 20 µl of the test compound is used herein according to the method of the present invention. More preferably, about 2 µl of the test compound is used herein.

In a preferred embodiment of the present invention about 20 µl of the sperm sample is added to the test solution and the test compound or the test solution and the test compound is added to about 20 µl of the sperm sample as defined elsewhere herein. More preferably, about 20 µl of the sperm sample is added to a volume of about 178 µl test solution and about 2 µl of the solvent used to prepare the test compound solution (vehicle control such as DMSO) adding up to a total volume of about 200 µl which refers to the control solution, or a volume of about 178 µl test solution and about 2 µl of the solvent used to prepare the test compound solution (vehicle control such as DMSO) is added to about 20 µl of the sperm sample as defined elsewhere herein. Also more preferably is that about 20 µl of the sperm sample is added to a volume of about 178 µl test solution and about 2 µl of the test compound adding up again to a total volume of about 200 µl which refers to the test solution, or that a volume of about 178 µl test solution and about 2 µl of the test compound is added to about 20 µl of the sperm sample as defined elsewhere herein. When referring to the preferred embodiment of the present invention, also about 20 µl of the sperm sample is added to the Ca²⁺-containing solution or vice versa as defined elsewhere herein, preferably using about 180 µl Ca²⁺-containing solution adding up again to a volume of 200 µl. Here, the test and control solutions are both essentially free of free-calcium (or other divalent ions as defined elsewhere herein) and differ mainly in the test compound added to the test solution, but not to the control solution. Accordingly, the control solution as used herein refers to a solution that is essentially free of free calcium (also preferably free of any other divalent ions such as magnesium), but without an added test compound (see **Fig. 1**).

After adding the sperm sample, preferably about 20 µl into a tube (or vial, or well of a multiwell assay plate) comprising the test solution and the test compound or vice versa as defined elsewhere herein, the tube (or vial, or well of a multi-well assay plate) then comprising the sperm sample, the test solution and the test compound and/or the tube (or vial, or well of a multi-well assay plate) then comprising the sperm sample and the control solution (without the test compound) may be gently mixed by inverting and/or low-speed vortexing and/or shaking and/or gentle repeated up-and-down pipetting.

The present invention also envisages that according to the method of identifying modulators of the CatSper channel activity described herein, the sperm sample is contacted for at least about 5 minutes to at most about 30 minutes with the test solution and the test compound in step (a) as defined elsewhere herein, preferably the sperm sample is contacted for about 15 minutes. Thus, the sperm cells comprised in a sperm sample may be contacted with the test solution and the test compound in step (a) as defined elsewhere herein for at least about 5 minutes, about 6 minutes, about 7 minutes, about 8 minutes, about 9 minutes, about 10 minutes, about 11 minutes, about 12 minutes, about 13 minutes, about 14 minutes, about 15 minutes, about 16 minutes, about 17 minutes, about 18 minutes, about 19 minutes, about 20 minutes, about 21 minutes, about 22 minutes, about 23 minutes, about 24 minutes, about 25 minutes, about 26 minutes, about 27 minutes, about 28 minutes, about 29 minutes, about 30 minutes. However, in case the test compound may be a CatSper activator the motility of the sperm cells can already be significantly affected after being contacted for about 5 minutes. In case the test compound is a CatSper inhibitor the motility of the sperm cells can already be significantly affected after being contacted for about 15 minutes. In a preferred embodiment of the present invention, the sperm sample is contacted for at least about 5 minutes to at most about 30 minutes with the test solution and the test compound of step (a) at 37 °C before detecting the motility of the sperm cells in step (b) of the herein described method. After the above described contacting time, the sperm sample contacted with the test solution and the test compound and the sperm sample contacted with the control solution may be gently mixed by inverting and/or low-speed vortexing and/or shaking and/or gentle repeated up-and-down pipetting before detecting the sperm cell motility as defined herein.

The term "contacted / "contacting" may also refer to "incubate" / "incubating". Preferably, "incubate" or "incubating" means incubating and/or gently mixing such as inverting and/or low-speed vortexing.

Throughout the present invention, the term "sperm" / "sperm cells" or "spermatozoa" refers to the plurality of more than one sperm cell (one spermatozoon). Such a plurality of more than one sperm cell may also be termed sperm cells / spermatozoa population or fraction of sperm cells / spermatozoa. In particular, a "fraction of sperm cells / spermatozoa" refers to a group of sperm cells comprised within a sperm sample. Sperm cells preferably used for the method of the present invention are obtained from a male subject with normozoospermia according to the guidelines of the WHO (WHO laboratory manual for the examination and processing of human semen) which may classify sperm count, sperm concentration, sample quality (pH, viscosity etc.) and sperm morphology to determine normozoospermia.

In this context, a "sperm sample" refers to a sample comprising sperm cells and seminal fluid with physiological amounts of organic and inorganic components. Such components of the seminal fluid may be citric acid, free amino acids, fructose, enzymes, phosphorylcholine, prostaglandin, potassium, and zinc. Such a sperm sample comprising sperm cells can be used without further treatment for the method of the present invention as defined elsewhere herein. However, the sperm sample may be pooled together with other sperm samples or may be diluted in a suitable buffer. In case such a preparation is needed, it takes less than about 5 minutes. Such a sperm sample may be obtained from a male subject by any method known to those skilled in the art.

The term "male subject" as used herein and throughout the present invention, also addressed as a male individual, refers preferably to a vertebrate, more preferably a mammal. Even more preferably, the mammal is any one of a human, a cattle, a buffalo, a horse, a donkey, a camel, a sheep, a goat, a pig, a dog, a cat, a rat, or a mouse, most preferably a human.

Preferably said sperm sample is obtained from said male subject by an ejaculation. A so called ejaculate refers to the orgasmic effusion of a male adolescent or sexually mature male subject comprising spermatozoa / sperm cells. It is preferred that sperm used for the present invention obtained from a male subject by ejaculation does not contain about 90% or more, such as about 91% or more, about 92% or more, about 93% or more, about 94% or more, about 95% or more, about 96% or more, about 97% or more, about 98% or more, about 99% or more or even about 100% motionless spermatozoa. Accordingly, sperm obtained from a male subject by ejaculation and containing larger amounts of motionless sperm cells, such as about 90% or more, such as about 91% or more, about 92% or more, about 93% or more, about 94% or more, about 95% or more, about 96% or more, about 97% or more, about 98% or more, about 99% or more or even about 100% motionless sperm cells are excluded from the method of the present invention. In other words, such particular sperm samples, which have a large amount of initially motionless spermatozoa, i.e. before the actual start of the test, cannot be and are not used for the method of the present invention. Accordingly, the sperm sample being contacted with the test solution and the test compound in step (a) of the method as described elsewhere herein comprises at least about 10% or more, such as about 15% or more, about 20% or more, about 30% or more, about 40% or more, about 50% or more, about 60% or more, about 70% or more, about 80% or more, about 90% or more or about 95 % or more motile sperm.

### Step b) of the method of the present invention

The method of the present invention of identifying modulators of the CatSper ion channel activity necessarily comprise as a second step (b) detecting the motility of each sperm cell in said sample under the test condition of step (a).

As used throughout the description, the terms "detect" / "detecting" include variations like "determine" or "determining", i.e. these terms may be used interchangeably. According to the present invention, the terms determination / detection are generally understood to refer to the motility of each sperm cell / motility of the sperm cells / motility of a fraction of sperm cells after being contacted with the control solution and/or the test solution comprising the test compound.

In this context, the term "detecting the motility" refers to determining whether the sperm cell in the sample is motile or immotile as defined elsewhere herein by using any technique for distinguishing between motile and immotile sperm cells. Thus, the term "determining the motility" may also be used interchangeably with the term detecting the motility. Preferably, when detecting / determining whether the sperm cell in the sperm sample is motile or immotile, or when the motility of the sperm cell is determined as used herein, any method selected from the group consisting of light microscopy, differential-dynamic microscopy, videomicrography, and methods detecting light-scattering (such as FACS) can be used. More preferably, when detecting / determining whether the sperm cell in the sperm sample is motile or immotile or the motility of the sperm cell is determined, light microscopy is used by applying any light microscope known to the person skilled in the art.

In case light microscopy is used, either numerous sperm cells are recorded at the same time with a sufficiently large microscopic image section (acquisition of successive individual images during the observation period), or many individual sperm cells are analyzed one after the other over a short period of time. The motility of the sperm cell is preferably determined manually or automatically. A manual determination or determining manually comprises either live imaging of the cells or taking microscopic pictures over a suitable amount of time. In case live imaging is used each sperm cell is observed for a few seconds to assess whether a forward movement can be determined or not. In case, microscopic pictures are taken, they may be taken over a suitable time frame in a suitable time to each other. Preferably, the suitable time is a fraction of a second. The resulting pictures can be analyzed manually by tracking single sperm cells through the pictures taken a fraction of a second from each other. Tracking by counting is only reliable if the motile sperm cover a distance in the time interval between two single images that is small enough to determine their "identity" from the previous image. Manually determining the motility comprises that at least about 10 sperm cells are analyzed by live imaging or taking microscopic pictures. The results of the motility of these sperm cells is then used to identify the test compound as CatSper modulator as defined elsewhere herein.

Thus, it is encompassed that manually determining the motility of each sperm cell may comprise counting of at least about 10 sperm cells of the sperm sample under the conditions of step (b). Thus, determining the motility of said sperm cell manually refers to counting of at least about 10, at least about 20, at least about 30, at least about 40, at least about 50, at least about 60, at least about 70, at least about 80, at least about 90, at least about 100, at least about 110, at least about 120, at least about 130, at least about 140, at least about 150, at least about 160, at least about 170, at least about 180, at least about 190, at least about 200, at least about 210, at least about 220, at least about 230, at least about 240, at least about 250, at least about 260, at least about 270, at least about 280, at least about 290, at least about 300, at least about 350, at least about 400, at least about 450, or at least about 500, preferably about 10, about 20, about 30, about 40, about 50, about 60, about 70, about 80, about 90, about 100, about 110, about 120, about 130, about 140, about 150, about 160, about 170, about 180, about 190, about 200, about 210, about 220, about 230, about 240, about 250, about 260, about 270, about 280, about 290, about 300, about 350, about 400, about 450, about 500 or more sperm cells of the sperm sample under the test condition of step (b), for example by using a light microscope. Preferably, determining the motility of each sperm cell manually, in other words determining the amount of motile sperm cells manually, comprises counting of at least about 10 sperm cells under the test conditions of step (b), for example by using a light microscope.

Determining the motility of said sperm cells automatically refers to taking microscope pictures of the sperm cells under the conditions of step (b) at different time points after the sperm sample has been contacted with the test solution and the test compound, additionally applying a particle tracking algorithm to these pictures. Such a particle tracking algorithm may be based on an algorithm developed by Crocker and Grier 1996. It usually determines the position of individual sperm cells in each picture taken at different time points and links the different positions of individual sperm cells over time; thereby it creates tracks of individual sperm cells and optionally classifies the amount of motile sperm cells according to the created tracks of individual sperm cells. In other words, a particle tracking software would be able to identify sperm cells in the first recorded image of the series, track the individual sperm cell over a time period image by image, and evaluate for each individual sperm cell whether it is stationary (= immotile, no forward movement as defined elsewhere herein) or moves independently forward as defined by the present invention (= motile).

When the motility of said sperm is determined automatically, light microscope may be used. In this context, the term "light microscope" may comprise an automated computer assisted sperm movement analysis called CASA, which is known to a person skilled in the art. Therefore, the present invention may also comprise a method of the present invention, wherein the motility of each sperm cell may be determined / detected automatically in step (b) by using CASA.

Alternatively, instead of evaluating single sperm cells by counting of at least about 10 sperm cells under the test conditions of step (b), also whole sperm populations may be evaluated, preferably using image fluctuation. To this end the spatiotemporal fluctuations of the intensity in time-lapse images from a sample are analyzed. These fluctuations directly correlate with the mean motility of the sample.

As used throughput the present invention, the term "motionless" used in combination with sperm cells refers to "immotile" sperm, meaning when a sperm is called "motionless" or "immotile" it does not move forward. It is encompassed that the motility of each sperm cell is characterized by any forward movement in comparison to immotility of a sperm cell characterized by no forward movement. A motile sperm cell (motile sperm) or as used in the present invention the motility of said sperm cell however may be characterized by any spatial forward movement in comparison to a sperm cell without spatial forward movement as used throughout the description. In other words, any forward movement as being used herein means any detectable, residual forward movement of the sperm cell resulting in a change of the spatial position. When a sperm cell is immotile according to the present invention, or immotility of the sperm cell is characterized, it refers to no detectable, residual forward movement resulting in a no spatial disposition. Such a special disposition may be only a few micrometers.

As known to the skilled person in the art the tail of the sperm is also called the flagellum, being the longest part and capable of wave-like motion that propels sperm for swimming and aids in the egg penetration process. In general, sperm motility depends on the 4 parts of the tail: connecting piece, midpiece, principal piece, and the end piece. The head of a sperm cell contains the nucleus and is surrounded by a thin, flattened sac called acrosome comprising enzymes being used for penetrating the female egg. In sum, any kind of independent / autonomous forward movement of the sperm cell, when the motility of the sperm cell in the sample being used in the method of the present invention is detected, may be a sufficient requirement for assigning (a) sperm cell (sperm) to the category of (a) motile sperm cell (sperm).

### Step c) of the method of the present invention

The method of the present invention of identifying a modulator of CatSper ion channel activity comprise as a third step (c) identifying the test compound as a modulator of CatSper channel activity if the presence of the compound is correlated with an altered motility of the sperm cell relative to the motility of the sperm cell in the test solution without the test compound.

As defined elsewhere herein, motility is any forward movement resulting in a spatial disposition. The term "relative to" as used herein means a comparison such as "in comparison with", in particular a comparison of the fraction of forward-moving sperm cells versus immotile sperm cells in the control solution with the fraction of forward-moving sperm cells versus immotile sperm cells in the test solution with the test compound. Thus, the decisive factor is how many sperm cells move and how many do not move. How far they move does not matter. Meaning each sperm cell that is detected in the control solution as defined herein and determined as being forward-moving or not as defined elsewhere herein may refer to a fraction of forward-moving versus immotile sperm cells; and each sperm cell that is detected in the test solution with the test compound as defined herein and determined as being forward-moving or not as defined elsewhere herein may also refer to another fraction of forward-moving versus immotile sperm cells. Those two fractions of forward-moving sperm cells versus immotile sperm cells (of the control and of the test solution) may then be compared as defined herein.

In particular this means, that if the presence of the test compound is correlated with a decreased motility of the sperm cell relative to the motility of the sperm cell in the test solution without the test compound, it is indicative that the test compound is a modulator capable of activating CatSper channel activity. A solution essentially free of free calcium inhibits the natural sperm movement / motility and the detected motility decreases over time (see **Fig. 1**). However, if the sperm sample is contacted with a test compound and the test solution and then the sperm cells loose their motility (any forward movement) faster than sperm cells in the control solution (also essentially free of free calcium, but not the test compound), it is a sign / it is indicative that such test compound is a modulator capable of activating CatSper channel activity (see **Fig. 1**). A decreased motility means in this context that a fraction of sperm cells in the test solution which is essentially free of free calcium and where the test compound has been added, has fewer forward-moving sperm cells (more immotile sperm cells) in comparison to the fraction of sperm cells in the control solution. In other words, decreased motility means that there is a decrease in the fraction of motile sperm cells in the test solution relative to the fraction of motile sperm cells in the control solution. In sum, in case a decrease in the motility of the sperm cells is detected, the modulator of the CatSper ion channel may be determined as an activator of the CatSper channel.

If, in another scenario, the presence of the compound is correlated with an increased motility of the sperm cell relative to the motility of the sperm cell in the test solution without the test compound, it is indicative that the test compound is a modulator capable of inhibiting CatSper channel activity. An increased motility means in this context that a fraction of sperm cells in the test solution and where the test compound has been added, has more forward-moving sperm cells (less immotile sperm cells) in comparison to the fraction of sperm cells in the control solution. In other words, increased motility means that there is an increase in the fraction of motile sperm cells in the test solution relative to the fraction of motile sperm cells in the control solution. In sum, in case an increase in the motility of the sperm cells is detected, the modulator of the CatSper ion channel is determined as an inhibitor of the CatSper channel.

Accordingly, if the presence of the compound is correlated with no difference in the motility of the sperm cell relative to the motility of a sperm cell in the test solution without the test compound, it is indicative that the test compound is not a modulator of CatSper channel activity. No difference in the motility means that there is neither an increase nor a decrease in the fraction of motile sperm cells in the test solution in comparison to the fraction of motile sperm cells in the control solution.

When detecting the motility of the sperm cells under the test condition of step (a), there may be a significant difference in the amount/fraction of motile sperm cells in the test solution and the control solution, which may be indicative for a test compound being a modulator of the CatSper ion channel activity. Thus, an increase and/or a decrease of the fraction of motile sperm cells in the test solution with a respective test compound is/are preferably significant in comparison to the fraction of motile sperm cells in the control solution. If there is no significant increase or decrease of the fraction of motile sperm cells in the test solution with a respective test compound in comparison to the fraction of motile sperm cells in the control solution, it is indicative that the test compound is not a modulator of CatSper channel activity. In other words, if the presence of the test compound is correlated with a decreased/increased motility of the sperm cell relative to the motility of the sperm cell in the test solution without the test compound and said increase/decrease of the fraction of motile sperm cells is significant in comparison to the fraction of motile sperm cells in the control solution, it is indicative that the test compound is a modulator capable of activating/inhibiting CatSper channel activity. To determine a significant difference between the fraction of motile sperm cells in the control solution and in the test solution, the motility of the sperm cells is preferably detected after at least about 5 minutes up to about 30 minutes. After 5 minutes an activator of the CatSper ion channel will significantly change the fraction of the motile sperm cells, while an inhibitor of the CatSper ion channel will need about 15 minutes to change the fraction of motile sperm cells significantly in comparison to the fraction of motile sperm cells in the control solution (see Fig. 1). A significant difference is to be understood as a mathematical significance, whereby a low significance level is sufficient representing about 90 % confidence interval.

Thus, the term "no significant change in the fraction of motile sperm cells" means that the fraction of motile sperm cells may not remarkably decrease / increase or that the fraction of motile sperm cells may remain more or less the same when the sperm sample obtained form said male subject is contacted with the calcium-free test solution and the test compound. In other words, when the term "no significant change in the fraction of motile sperm cells" is used, it means that the fraction of motile sperm cells treated with the test solution and the test compound does not decrease / increase by more than about 10% in comparison to that treated with the control solution. It means that less than about 10% or less, about 9% or less, about 8% or less, about 7% or less, about 6% or less, about 5% or less, about 4% or less, about 3% or less, about 2% or less, about 1% or less, or even 0% of the sperm cells either display or do not display forward movement when the motility of the sperm cells in said sample has been detected under the test conditions of step (a) in comparison to the motility of sperm cells treated with the control solution.

Further, the method of the present invention can also be used to evaluate the quality of said modulator such as an inhibitor or activator identified of the CatSper ion channel activity. Thus, not only the method of the invention is used to identify a modulator of CatSper channel activity as defined elsewhere herein, but also the quality of said modulator may be analyzed/determined afterwards. As described above, the fraction of motile sperm cells over time in the control solution results in a gradual decreasing curve over time (see Fig. 2 - 0µM of the known inhibitor). By comparing the steepness of the curve per time resulting from a sperm sample being contacted with the test solution without the test compound (= control solution) with the steepness of a curve per time resulting from the sperm sample being contacted with the test test solution and the test compound, the quality or efficacy of the test compound can be determined.

In theory, the quality or efficacy of a CatSper modulator can also be determined via an one-point measurement at a point in time at which the two curves displaying the motility of the sperm cells treated with (potent) inhibitor / activator are as far apart as possible, compared to sperm cells in the control solution (see also **Fig. 2**).

In more detail, the fractional motility curve of sperm contacted with a potent CatSper inhibitor should fall less steep in comparison to the curve of the motility of sperm cells contacted with a less potent CatSper inhibitor. This is also exemplified in **Figure 2****,** which displays three sperm motility curves contacted with different concentrations of a potent CatSper inhibitor (3, 6 and 12 µM). All curves differ from each other. The curve displaying the motility of sperm cells treated with the highest concentration (12 µM) is less steep in comparison to the control solution (0 µM of the known inhibitor), e.g. reaches a motility of about 116 % after about 10 minutes. Sperm cells in the Ca²⁺-free control solution display, in comparison to that, a motility of only about 23 % after about 10 minutes. As example for a less potent inhibitor, the concentrations of 3 and 6 µM, respectively, are shown in **Figure. 2****.** These curves reach a motility of about 106 and 110 %, respectively, after about 10 minutes. In sum, the more potent/powerful an inhibitor is, the less steep the curve will be compared to the curve of the control solution without the inhibitor.

As mentioned elsewhere herein, a calcium flux occurs through said CatSper ion channel along the concentration gradient of the intra- and extracellular calcium concentration. CatSper-deficient sperm, however, lack the exit port for Ca²⁺-ions once a decrease in the extracellular calcium concentration occurs (as mentioned elsewhere herein); their motility is conserved under calcium-free conditions over an extended period of time. Comparing the ratio of motile versus immotile sperm cells in a calcium-free test solution, which has been contacted with the sperm sample used in the present invention may be used to determine whether sperm cells have functional CatSper channels. If the sperm cells remain motile for a long time, there may be a CatSper defect or other serious defects in the calcium-dependent signaling process, leading to the conclusion that the male subject may be infertile. If the sperm cells are / become immotile, the CatSper channel is most likely functional.

Meaning, if the sperm sample is contacted with the calcium-free test solution and with and without a test compound as defined in step (a) of the method of identifying modulators of the CatSper ion channel activity and the amount / fraction of motile sperm cells in the test solution with a test compound is significantly different in comparison to the sperm cells in the control solution as defined elsewhere herein, thus identifying said test compound as a CatSper inhibitor, the test compound may be used as a contraceptive.

To initially determine the motility of the sperm cells of a sperm sample, the inventors compared the motility of the sperm cells under the test conditions with the motility of the sperm cells in a calcium-containing solution, which corresponds regarding the ionic composition to e.g. natural oviductal fluid (see **Fig. 2**). The inventors contacted the sperm sample with a calcium-free test / control solution and a calcium-containing solution to demonstrate the impact of the calcium-free conditions on the motility of the sperm cells in said sperm sample (see **Fig. 2**).

As depicted in **Table 1,** the term "calcium-containing solution" refers to a solution comprising more than about 1000 nM, more than about 10.000 nM, more than about 100.000 nM, more than about 1 mM free calcium. It may correspond to a HTF-buffer (human tubular fluid) as known to the skilled person.

With regard to the quality of a test compound being identified as a potent CatSper activator, the fractional motility curve of sperm contacted with a potent CatSper activator should fall steeper with increasing potency / efficiency of said activator in comparison to the curve of the motility of sperm cells contacted with a less potent CatSper activator. In sum, the more potent/powerful an activator is, the steeper the curve should fall compared to the curve of the control solution without the activator.

It is noted that as used herein, the singular forms "a", "an", and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein.

Unless otherwise indicated, the term "at least" preceding a series of elements is to be understood to refer to every element in the series. The term "at least one" refers to one or more such as two, three, four, five, six, seven, eight, nine, ten or more.

The term "and/or" wherever used herein includes the meaning of "and", "or" and "all or any other combination of the elements connected by said term".

The term "less than" or in turn "more than" does not include the concrete number.

For example, less than 20 mean less than the indicated number. Similarly, more than or greater than means more than or greater than the indicated number, e.g. more than 80 % means more than or greater than the indicated number of 80 %.

Throughout this specification and the claims that follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having". When used herein "consisting of" excludes any element, step, or ingredient not specified.

The term "including" means "including but not limited to". "Including" and "including but not limited to" are used interchangeably.

The term "about" means plus or minus 10%, preferably plus or minus 5%, more preferably plus or minus 2%, most preferably plus or minus 1%.

Throughout the description and claims of this specification, the singular encompasses the plural unless the context otherwise requires. In particular, where the indefinite article is used, the specification is to be understood as contemplating plurality as well as singularity, unless the context requires otherwise.

It should be understood that this invention is not limited to the particular methodology, protocols, material, reagents, and substances, etc., described herein and as such can vary. The terminology used herein is for the purpose of describing particular embodiments only, and is not intended to limit the scope of the present invention, which is defined solely by the claims.

A better understanding of the present invention and of its advantages will be gained from the following examples, offered for illustrative purposes only. The examples are not intended to limit the scope of the present invention in any way.

### EXAMPLES OF THE INVENTION

### Example 1: CatSper modulator screening

### Materials used in the present invention:

The Ca²⁺-containing solution being used in **Figure 1** of the present invention corresponds to the HTF buffer (human tubular fluid) used for years in research with spermatozoa (Strünker et al. 2011, Nature 471, 382-386). The calcium-free solution is also based on this buffer. It is very likely that the test can also be carried out with buffer solutions of a different composition, as long as they are kept in the physiological range and comprise the herein mentioned ranges or values for calcium and magnesium.

**Table 1: Composition of the Ca²⁺-containing and the Ca²⁺-free solutions being used in the method of the present invention.**

| | **Calcium-containing solution** | **Calcium-free solution** |
|---|---|---|
| NaCl [mM] | 93,8 | 93,8 |
| KCl [mM] | 4,7 | 4,7 |
| MgSO₄ [mM] | 0,2 | 0,2 |
| KH₂PO₄ [mM] | 0,37 | 0,37 |
| CaCl₂ [mM] | 2,04 | 0 |
| HEPES [mM] | 21 | 21 |
| Glucose [mM] | 2,78 | 2,78 |
| Na-Lactate [mM] | 21,4 | 21,4 |
| NaHCO₃ [mM] | 4 | 4 |
| EGTA [mM] | 0 | 5 |
| Pyruvate [mM] | 0,33 | 0,33 |
| Human serum albumin [mg/ml] | 3 | 3 |

For performing a CatSper-Screening-Test, 178 µl of the calcium-free solution and 2 µl of the solvent used to prepare the test compound solution is used to produce the control solution (calcium-free solution without any test compound). A test solution is produced by adding 178 µl of the calcium-free solution and 2 µl of the test compound solution together. Optionally, another 180 µl of the calcium-containing solution (without any test compound) may further be used in another vial (see particular composition above in **Tab. 1**). The solutions are then stored at 4 °C and heated to 37 °C before use to warm up the solutions.

A spermatozoa (semen) sample is then obtained from a male subject who is a healthy volunteer. A volume of 20 µl of the ejaculate is added to the calcium-free control, calcium-free test and calcium-containing solutions in each micro reaction tube. By low-speed vortex mixing or repeatedly inverting the micro reaction tubes, a homogeneous spermatozoa suspension is generated. Both micro reaction tubes are then incubated at 37 °C for 15 min. After incubation, the mixing step is repeated and the spermatozoa motility is determined for the conditions by counting a total number of 200 sperm cells. The fraction of motile sperm is then compared for the calcium-free control solution with and without any test compound. If a difference in the fraction of motile sperm is determined the test compound is identified as inhibitor / activator or no modulator of the CatSper activity.

For example, in the presence of the RU1968 inhibitor (30 µM), sperm cells from healthy volunteers behave in the same way as sperm from patients with CatSper dysfunction. The inventors can therefore cite RU1968 as a proof-of-principal CatSper inhibitor. Progesterone (10 µM) was used as a test compound which activates CatSper ion channel activity and is therefore considered as a proof-of-principal activator of said ion channel (see **Fig. 1**).

This experiment demonstrates that the screening test of the present invention is suitable as a phenotypic screening method for testing test compounds as described elsewhere herein in search of CatSper modulators.

## Claims

1. A method of identifying a modulator of CatSper channel activity, comprising the steps of:
a) contacting a sperm sample with a test solution that is essentially free of free calcium, wherein the test solution that is essentially free of free calcium comprises less than about 20 nM free calcium, and a test compound:
b) detecting the motility of each sperm cell in the sample under the test condition of step (a);
c) identifying the test compound as a modulator of CatSper channel activity if the presence of the compound is correlated with an altered motility of the sperm cell relative to the motility of the sperm cell in the test solution without the test compound.

2. The method of claim 1, wherein the sperm sample is contacted for at least about 5 minutes to at most about 30 minutes with the test solution and the test compound in step (a), preferably the sample is contacted for about 15 minutes.

3. The method of claim 1 or 2, wherein if the presence of the test compound is correlated with a decreased motility of the sperm cell relative to the motility of the sperm cell in the test solution without the test compound, it is indicative that the test compound is a modulator capable of activating CatSper channel activity.

4. The method of claim 1 or 2, wherein if the presence of the compound is correlated with an increased motility of the sperm cell relative to the motility of the sperm cell in the test solution without the test compound, it is indicative that the test compound is a modulator capable of inhibiting CatSper channel activity.

5. The method of claim 1 or 2, wherein if the presence of the compound is correlated with no difference in the motility of the sperm cell relative to the motility of the sperm cell in the test solution without the test compound, it is indicative that the test compound is not a modulator of CatSper channel activity.

6. The method of any one of the preceding claims, wherein the test solution that is essentially free of free calcium comprises at least one agent reducing the free calcium concentration, wherein the at least one agent reducing the free calcium concentration is a chemical capable of binding Ca²⁺, optionally wherein the chemical capable of binding Ca²⁺ is any one of ethylene glycol-bis(β-aminoethyl ether)-*N*,*N*,*N'*,*N'*-tetraacetic acid (EGTA), ethylenediaminetetraacetic acid (EDTA), 1,2-bis(*o*-aminophenoxy)ethane-*N*,*N*,*N'*,*N'*-tetraacetic acid (BAPTA), *N*-(2-Hydroxyethyl)ethylenediamine-*N*,*N'*,*N'-*triacetic acid (HEDTA), nitrilotriacetic acid (NTA), dibromo-1,2,-bis(o-aminophenoxy)ethane-N,N,-N',N',tetraacetic acid (DiBrBAPTA), citric acid, gluconate solution, aspartate solution, phosphate, or a Ca²⁺-indicator dye, preferably EGTA.

7. The method of any one of the preceding claims, wherein the test solution is further essentially free of free magnesium, wherein the test solution that is further essentially free of free magnesium comprises less than about 250 µM free magnesium.

8. The method of any one of the preceding claims, wherein the test compound is any one of a ligand, a chemical compound, an aptamer, or a lipid or a combination thereof, optionally wherein the ligand is any one of a chemical compound, a peptide, or a protein or a combination thereof.

9. The method of any one of the preceding claims, wherein the sperm sample is contacted simultaneously with the test solution and the test compound.

10. The method of any one of the preceding claims, wherein the motility of each sperm cell is **characterized by** any forward movement in comparison to immotility of a sperm cell **characterized by** no forward movement.

11. The method of any one of the preceding claims, wherein the motility of each sperm cell is determined by using any method selected from the group consisting of light microscopy, differential dynamic microscopy, videography and methods detecting light scattering, optionally wherein the motility of each sperm cell is determined using light microscopy.

12. The method of any one of the preceding claims, wherein the motility of each sperm cell is determined manually or automatically, optionally wherein manually determining the motility of each sperm cell comprises counting of at least about 10 sperm cells of the sperm sample under the conditions of step (b).

13. The method of any one of the preceding claims, wherein the sperm sample is obtained from a male subject.

14. The method of claim 13, wherein the male subject is a vertebrate, optionally wherein the vertebrate is a mammal.

15. The method of claim 14, wherein the mammal is any one of a human, a cow, a buffalo, a horse, a donkey, a camel, a sheep, a goat, a pig, a dog, a cat, a rat, or a mouse, preferably a human.

## Patentansprüche

1. Verfahren zur Identifizierung eines Modulators der Aktivität des CatSper-Kanals, das die folgenden Schritte umfasst:
a) in Kontakt bringen einer Spermaprobe mit einer Testlösung, die im Wesentlichen frei von freiem Calcium ist, wobei die Testlösung, die im Wesentlichen frei von freiem Calcium ist, weniger als etwa 20 nM freies Calcium und einer Testverbindung umfasst;
b) Nachweis der Motilität jeder Samenzelle in der Probe unter den Testbedingungen von Schritt (a);
c) Identifizieren der Testverbindung als Modulator der CatSper-Kanalaktivität, wenn das Vorhandensein der Verbindung mit einer veränderten Motilität der Spermazelle im Vergleich zur Motilität der Spermazelle in der Testlösung ohne die Testverbindung korreliert.

2. Verfahren nach Anspruch 1, wobei die Spermaprobe für mindestens etwa 5 Minuten bis höchstens etwa 30 Minuten lang mit der Testlösung und der Testverbindung in Schritt (a) in Kontakt gebracht wird, vorzugsweise wird die Probe etwa 15 Minuten lang in Kontakt gebracht.

3. Verfahren nach Anspruch 1 oder 2, wobei, wenn das Vorhandensein der Testverbindung mit einer verringerten Motilität der Spermazelle im Vergleich zur Motilität der Spermazelle in der Testlösung ohne die Testverbindung korreliert, dies ein Hinweis darauf ist, dass die Testverbindung ein Modulator ist, der die Aktivität des CatSper-Kanals aktivieren kann.

4. Verfahren nach Anspruch 1 oder 2, wobei, wenn das Vorhandensein der Verbindung mit einer erhöhten Motilität der Spermazelle im Vergleich zur Motilität der Spermazelle in der Testlösung ohne die Testverbindung korreliert, dies ein Hinweis darauf ist, dass die Testverbindung ein Modulator ist, der die Aktivität des CatSper-Kanals hemmen kann.

5. Verfahren nach Anspruch 1 oder 2, wobei, wenn das Vorhandensein der Verbindung mit keinem Unterschied in der Motilität der Spermazelle im Vergleich zur Motilität der Spermazelle in der Testlösung ohne die Testverbindung korreliert, dies ein Hinweis darauf ist, dass die Testverbindung kein Modulator der CatSper-Kanalaktivität ist.

6. Verfahren nach einem der vorhergehenden Ansprüche, wobei die Testlösung, die im Wesentlichen frei von freiem Calcium ist, mindestens ein Mittel umfasst, das die Konzentration an freiem Calcium reduziert, wobei das mindestens eine Mittel, das die Konzentration an freiem Calcium reduziert, eine Chemikalie ist, die in der Lage ist, Ca²⁺ zu binden, optional wobei die Chemikalie, die in der Lage ist, Ca²⁺ zu binden, eine der folgenden ist: Ethylenglycol-bis(β-Aminoethylether)-*N*,*N*,*N'*,*N'*-Tetraessigsäure (EGTA), Ethylendiamintetraessigsäure (EDTA), 1,2-Bis(*o*-Aminophenoxy)ethan-*N,N,N',N'*-Tetraessigsäure (BAPTA), *N*-(2-Hydroxyethyl)ethylendiamin-*N,N',N'-*Triessigsäure (HEDTA), Nitrilotriessigsäure (NTA), Dibromo-1,2,-bis(o-Aminophenoxy)ethan-N,N,-N',N',Tetraessigsäure (DiBrBAPTA), Zitronensäure, Gluconatlösung, Aspartatlösung, Phosphat oder ein Ca²⁺-Indikatorfarbstoff, vorzugsweise EGTA.

7. Verfahren nach einem der vorhergehenden Ansprüche, wobei die Testlösung weiterhin im Wesentlichen frei von freiem Magnesium ist, wobei die Testlösung, die weiterhin im Wesentlichen frei von freiem Magnesium ist, weniger als etwa 250 µM freies Magnesium umfasst.

8. Verfahren nach einem der vorhergehenden Ansprüche, wobei die Testverbindung ein Ligand, eine chemische Verbindung, ein Aptamer oder ein Lipid oder eine Kombination davon ist, optional wobei der Ligand eine chemische Verbindung, ein Peptid oder ein Protein oder eine Kombination davon ist.

9. Verfahren nach einem der vorhergehenden Ansprüche, wobei die Spermaprobe gleichzeitig mit der Testlösung und der Testverbindung in Kontakt gebracht wird.

10. Verfahren nach einem der vorhergehenden Ansprüche, wobei die Motilität jeder Spermazelle durch jegliche Vorwärtsbewegung im Vergleich zur Unbeweglichkeit einer Spermazelle, die durch keine Vorwärtsbewegung gekennzeichnet ist, gekennzeichnet ist.

11. Verfahren nach einem der vorhergehenden Ansprüche, wobei die Motilität jeder Spermazelle bestimmt wird unter Verwendung eines beliebigen Verfahrens ausgewählt aus der Gruppe bestehend aus Lichtmikroskopie, dynamischer Differenzialmikroskopie, Videographie und Verfahren zum Nachweis von Lichtstreuung, optional wobei die Motilität jeder Spermazelle unter Verwendung von Lichtmikroskopie bestimmt wird.

12. Verfahren nach einem der vorhergehenden Ansprüche, wobei die Motilität jeder Spermazelle manuell oder automatisch bestimmt wird, optional wobei die manuelle Bestimmung der Motilität jeder Spermazelle das Zählen von mindestens etwa 10 Spermazellen der Spermaprobe unter den Bedingungen von Schritt (b) umfasst.

13. Verfahren nach einem der vorhergehenden Ansprüche, wobei die Spermaprobe von einem männlichen Subjekt erhalten wird.

14. Verfahren nach Anspruch 13, wobei das männliche Subjekt ein Wirbeltier ist, optional wobei das Wirbeltier ein Säugetier ist.

15. Verfahren nach Anspruch 14, wobei das Säugetier ein Mensch, eine Kuh, ein Büffel, ein Pferd, ein Esel, ein Kamel, ein Schaf, eine Ziege, ein Schwein, ein Hund, eine Katze, eine Ratte oder eine Maus ist, vorzugsweise ein Mensch.

## Revendications

1. Méthode d'identification d'un modulateur de l'activité du canal CatSper, comprenant les étapes suivantes
a) mettre en contact un échantillon de sperme avec une solution d'essai essentiellement exempte de calcium libre, dans laquelle la solution d'essai essentiellement exempte de calcium libre comprend moins d'environ 20 nM de calcium libre, et un composé d'essai;
b) détecter la mobilité de chaque spermatozoïde de l'échantillon dans les conditions de test de l'étape a);
c) identifier le composé d'essai comme modulateur de l'activité du canal CatSper si la présence du composé est corrélée à une modification de la mobilité du spermatozoïde par rapport à la mobilité du spermatozoïde dans la solution d'essai sans le composé d'essai.

2. La méthode de la revendication 1, dans laquelle l'échantillon de sperme est mis en contact pendant au moins 5 minutes et au plus 30 minutes avec la solution de test et le composé de test à l'étape (a), de préférence l'échantillon est mis en contact pendant environ 5 minutes.

3. La méthode de la revendication 1 ou 2, dans laquelle si la présence du composé d'essai est corrélée à une diminution de la motilité du spermatozoïde par rapport à la motilité du spermatozoïde dans la solution d'essai sans le composé d'essai, cela indique que le composé d'essai est un modulateur capable d'activer l'activité du canal CatSper.

4. La méthode de la revendication 1 ou 2, dans laquelle si la présence du composé est corrélée à une augmentation de la motilité du spermatozoïde par rapport à la motilité du spermatozoïde dans la solution d'essai sans le composé d'essai, cela indique que le composé d'essai est un modulateur capable d'inhiber l'activité du canal CatSper.

5. La méthode de la revendication 1 ou 2, dans laquelle si la présence du composé est corrélée à l'absence de différence dans la motilité du spermatozoïde par rapport à la motilité du spermatozoïde dans la solution d'essai sans le composé d'essai, cela indique que le composé d'essai n'est pas un modulateur de l'activité du canal CatSper.

6. La méthode de l'une quelconque des revendications précédentes, dans laquelle la solution d'essai qui est essentiellement exempte de calcium libre comprend au moins un agent réduisant la concentration de calcium libre, dans laquelle l'au moins un agent réduisant la concentration de calcium libre est un produit chimique capable de lier le Ca²⁺, éventuellement dans laquelle le produit chimique capable de lier le Ca²⁺ est l'un quelconque de l'éthylène glycol-bis(β-aminoéthyl éther)-*N,N,N',N'*-tétraacétique (EGTA), l'acide éthylènediaminetétraacétique (EDTA), l'acide 1,2-bis(*o*-aminophénoxy)éthane-*N,N,N',N'*-tétraacétique (BAPTA), l'acide *N*-(2-Hydroxyéthyl)éthylènediamine-*N,N',N'*-triacétique (HEDTA), l'acide nitrilotriacétique (NTA), l'acide dibromo-1,2,-bis(o-aminophénoxy)éthane-N,N,-N',N',tétraacétique (DiBrBAPTA), l'acide citrique, la solution de gluconate, la solution d'aspartate, le phosphate ou un colorant indicateur de Ca²⁺, de préférence l'EGTA.

7. La méthode de l'une des revendications précédentes, dans laquelle la solution d'essai est en outre essentiellement exempte de magnésium libre, la solution d'essai qui est en outre essentiellement exempte de magnésium libre comprenant moins d'environ 250 µM de magnésium libre.

8. La méthode de l'une des revendications précédentes, dans laquelle le composé de test est un ligand, un composé chimique, un aptamère ou un lipide ou une combinaison de ceux-ci, éventuellement dans laquelle le ligand est un composé chimique, un peptide ou une protéine ou une combinaison de ceux-ci.

9. La méthode de l'une des revendications précédentes, dans laquelle l'échantillon de sperme est mis en contact simultanément avec la solution de test et le composé de test.

10. La méthode de l'une des revendications précédentes, dans laquelle la motilité de chaque spermatozoïde est **caractérisée par** un mouvement vers l'avant par rapport à l'immotilité d'un spermatozoïde **caractérisé par** l'absence de mouvement vers l'avant.

11. La méthode de l'une des revendications précédentes, dans laquelle la motilité de chaque spermatozoïde est déterminée à l'aide d'une méthode choisie dans le groupe constitué de la microscopie optique, de la microscopie dynamique différentielle, de la vidéographie et des méthodes de détection de la diffusion de la lumière, éventuellement dans laquelle la motilité de chaque spermatozoïde est déterminée à l'aide de la microscopie optique.

12. La méthode de l'une des revendications précédentes, dans laquelle la motilité de chaque spermatozoïde est déterminée manuellement ou automatiquement, éventuellement dans laquelle la détermination manuelle de la motilité de chaque spermatozoïde comprend le comptage d'au moins environ 10 spermatozoïdes de l'échantillon de sperme dans les conditions de l'étape (b).

13. La méthode de l'une des revendications précédentes, dans laquelle l'échantillon de sperme est obtenu à partir d'un sujet masculin.

14. La méthode de la revendication 13, dans laquelle le sujet mâle est un vertébré, éventuellement dans laquelle le vertébré est un mammifère.

15. Méthode de la revendication 14, dans laquelle le mammifère est un humain, une vache, un buffle, un cheval, un âne, un chameau, un mouton, une chèvre, un porc, un chien, un chat, un rat ou une souris, de préférence un humain.
